# EUROPEAN PATENT APPLICATION

(11) **EP 4 397 229 A1**
(43) Date of publication of application: **10.07.2024**
(21) Application number: 23150088.5
(22) Date of filing: 03.01.2023
(51) Int. Cl.: A61B 5/00, A61B 5/055, A61B 6/00, G01R 33/567

(54) **COMPUTING DEVICE FOR PROVIDING TRIGGERING TIMES TO A MEDICAL SCANNING APPARATUS AND METHOD THEREOF**

(71) Applicant: Siemens Healthineers AG, 91301 Forchheim (DE)
(72) Inventor: Gadjimuradov, Fasil, 91054 Erlangen (DE); Hegde, Sanketa, 91054 Erlangen (DE); Krishnamurthy, Uday Bhaskar, St. Louis, 63146 (US); Vahle, Thomas, 90409 Nürnberg (DE)
(74) Representative: Siemens Healthineers Patent Attorneys

(57) **Abstract**

A computing device for providing triggering times to a medical scanning apparatus adapted to perform triggered imaging data acquisition, said computing device comprising an input data interface, configured to obtain real-time data of respiratory cycles of a patient; a computation module, configured to implement an artificial intelligence entity, which is trained and adapted to generate a prediction of a number of respiratory cycles of the patient based on the obtained real-time data; a triggering module, configured to determine triggering times corresponding to the predicted respiratory cycles; and an output data interface, configured to output the determined triggering times to the medical scanning apparatus.

## Description

Independent of the grammatical term usage, individuals with male, female or other gender identities are included within the term.

The present invention relates to a computing device for providing triggering times to a medical scanning apparatus, said medical scanning apparatus being configured to acquire triggered imaging data. The present invention further relates to a computer-implemented method for providing triggering times to such a scanning apparatus.

The invention is mostly described with respect to magnetic resonance imaging (MRI), but the principles of the invention have a broader scope and apply equally well to more general medical applications involving medical scanning apparatuses.

The acquisition of imaging data from patients in medical applications, for instance using magnetic resonance imaging (MRI), is still nowadays done by asking the patients to perform breath-holds during the exposition time. This mechanism to limit the body-motion gives generically the best image quality of the scans.

However, breath-holds can be especially challenging for a broad class of patients, including pediatric, geriatric, psychiatric or simply patients with respiratory conditions. Breath-holds in these cases can be unsuccessful, resulting in blurred scans, especially when the scans are taken around the thorax or the abdomen areas.

Several free-breathing techniques are known that aim at limiting the body motion caused by the breathing activity. Some of these techniques focus on extracting motion states from the acquired data during the scanning time. However, they typically require long exposition times, which can also be problematic for the patient groups described above. Moreover, since different motion states are in place, one has to deal with a large amount of data and a rather laborious image processing.

Triggered acquisitions, in contrast, are based on the principle of triggering the data acquisition close to the end-expiratory peak and/or end-inspiratory peak of the respiratory cycles, where the body motion caused by the breathing activity is minimal. Satisfactory triggered acquisitions rely on having reliable knowledge of the respiratory pattern of the patient and a suitable trigger algorithm, which can trigger the acquisition times of an MRI machine.

Conventional trigger algorithms use heuristic approaches to determine the triggering times that signal the beginning time of data acquisition to the medical scanner. In some of these trigger algorithms, there is an initial learning phase, in which the respiratory pattern of a patient is characterized by detecting a number of maxima and minima of the respiratory signal, i.e. the end-inspiration and end-expiration points, respectively, of the respiratory cycles. In a subsequent phase, a trigger threshold point is calculated as a function of the average maxima and minima detected during the learning phase. In some of these algorithms, this trigger threshold is updated regularly, typically at the conclusion of each respiratory cycle.

In case the respiratory signal goes below the trigger threshold, the trigger threshold is taken as a valid triggering time. The MRI machine is triggered to start the data acquisition for a predetermined time, after which it pauses the data acquisition and waits for the next trigger instruction.

It is an object of the present invention to provide a computing device for triggered data acquisition in medical free-breathing scans, which performs an enhanced determination of the scanning acquisition times through an improved calculation of the triggering times, and correspondingly optimizes the reduction of respiratory-associated motion during the scanning time.

The purpose of this invention is achieved through a device with the features disclosed in claim 1 and a computer-implemented method with the features detailed in claim 12. Preferred embodiments of the invention with advantageous features are given in the dependent claims.

Herein and in the forthcoming, independent of the grammatical term usage, individuals with male, female or other gender identities are included within the term.

A first aspect of the invention provides a computing device for providing triggering times to a medical scanning apparatus adapted to perform triggered imaging data acquisition, said computing device comprising an input data interface, configured to obtain real-time data of respiratory cycles of a patient; a computation module, configured to implement an artificial intelligence entity, which is adapted, in particular trained and adapted, to generate a prediction of a number of respiratory cycles of the patient based on the obtained real-time data; a triggering module, configured to determine triggering times corresponding to the predicted respiratory cycles; and an output data interface, configured to output the determined triggering times to the medical scanning apparatus.

Triggered imaging data acquisitions (or triggered scans) refer to data captured by different means (e.g. magnetic resonance) using some triggering mechanism (e.g. in the form of an activation signal), which can be processed, digitalized and visualized as an image.

A medical scanning apparatus (or medical scanner) is to be understood broadly as any device in the medical environment configured to generate scans, in particular triggered scans. An example of a medical scanning apparatus is a magnetic resonance imaging (MRI) scanner.

Triggering times are, in the context of the present invention, time-points where a medical scanner can be triggered to start acquiring data. Hereinafter triggering times will also be referred to as triggering points.

A respiratory cycle is to be understood as the minimum time interval between two periodic respiratory events, e.g. the time interval between two full expirations or the time interval between two full inspirations.

Real-time data of respiratory cycles of a patient refers to the data obtained from a patient through a number of sensors that acquire information about the breathing amplitude values over time immediately before and during a scanning session.

Herein and in the forthcoming, a predicted respiratory cycle or a predicted respiratory pattern or a predicted respiratory activity refer to a prediction of the breathing amplitude values over time.

Whenever herein an artificial intelligence entity is mentioned, it shall be understood as a computerized entity able to implement different data analysis methods broadly described under the terms artificial intelligence, machine learning, deep learning or computer learning. The artificial intelligence entity can be a multilayer perceptron network (MLP), a recurrent neural network (RNN), a long short-term memory (LSTM) network, a convolutional neural network (CNN) or any other neural network.

The different modules mentioned in this application are broadly understood as entities capable of acquiring, obtaining, receiving or retrieving generic data and/or instructions through a user interface and/or programming code and/or executable programs or any combination thereof. The different modules are adapted to run programming code and executable programs and to deliver the results for further processing.

The different modules, or parts thereof, may therefore each contain, at least, a central processing unit, CPU, and/or at least one graphics processing unit, GPU, and/or at least one field-programmable gate array, FPGA, and/or at least one application-specific integrated circuit, ASIC and/or any combination of the foregoing. Each of them may further comprise a working memory operatively connected to the at least one CPU and/or a non-transitory memory operatively connected to the at least one CPU and/or the working memory. Each of them may be implemented partially and/or completely in a local apparatus and/or partially and/or completely in a remote system such as by a cloud computing platform.

All of the elements of the computing device may be realized in hardware and/or software, cable-bound and/or wireless, and in any combination thereof. Any of the elements may comprise an interface to an intranet or the Internet, to a cloud computing service, to a remote server and/or the like.

In particular, the computing device of the invention may be implemented partially and/or completely in a local apparatus, e.g. a computer, in a system of computers and/or partially and/or completely in a remote system such as a cloud computing platform.

In systems based on cloud computing technology, a large number of devices is connected to a cloud computing system via the Internet. The devices may be located in a remote facility connected to the cloud computing system. For example, the devices can comprise, or consist of, equipment, sensors, actuators, robots, and/or machinery in an industrial set-up(s). The devices can be medical devices and equipment in a healthcare unit. The devices can be home appliances or office appliances in a residential/commercial establishment.

The cloud computing system may enable remote configuring, monitoring, controlling, and maintaining connected devices (also commonly known as 'assets'). Also, the cloud computing system may facilitate storing large amounts of data periodically gathered from the devices, analyzing the large amounts of data, and providing insights (e.g., Key Performance Indicators, Outliers) and alerts to operators, field engineers or owners of the devices via a graphical user interface (e.g., of web applications). The insights and alerts may enable controlling and maintaining the devices, leading to efficient and fail-safe operation of the devices. The cloud computing system may also enable modifying parameters associated with the devices and issues control commands via the graphical user interface based on the insights and alerts.

The cloud computing system may comprise a plurality of servers or processors (also known as 'cloud infrastructure'), which are geographically distributed and connected to each other via a network. A dedicated platform (hereinafter referred to as 'cloud computing platform') is installed on the servers/processors for providing above functionality as a service (hereinafter referred to as 'cloud service'). The cloud computing platform may comprise a plurality of software programs executed on one or more servers or processors of the cloud computing system to enable delivery of the requested service to the devices and its users.

One or more application programming interfaces (APIs) are deployed in the cloud computing system to deliver various cloud services to the users.

A second aspect of the present invention provides a computer-implemented method for providing triggering times to a medical scanning apparatus adapted to perform triggered imaging data acquisition, comprising the following steps: (a) obtaining real-time data of respiratory cycles of a patient; (b) generating, using an artificial intelligence entity, a prediction of a number of respiratory cycles of the patient based on the obtained real-time data; (c) determining triggering times corresponding to the predicted respiratory cycles; and (d) outputting the triggering times to the medical scanning apparatus.

In particular, the method according to the second aspect of the invention may be carried out by the computing device according to the first aspect of the invention. The features and advantages disclosed herein in connection with the computing device are therefore also disclosed for the method, and vice versa.

According to a third aspect, the invention provides a computer program product comprising executable program code configured to, when executed, perform the method according to the second aspect of the present invention.

According to a fourth aspect, the invention provides a non-transient computer-readable data storage medium comprising executable program code configured to, when executed, perform the method according to the second aspect of the present invention.

The non-transient computer-readable data storage medium may comprise, or consist of, any type of computer memory, in particular semiconductor memory such as a solid-state memory. The data storage medium may also comprise, or consist of, a CD, a DVD, a Blu-Ray-Disc, an USB memory stick or the like.

According to a fifth aspect, the invention provides a data stream comprising, or configured to generate, executable program code configured to, when executed, perform the method according to the second aspect of the present invention. According to a sixth aspect, the invention provides a medical scanning system, comprising the computing device according to the first aspect of the invention, and a medical scanning apparatus, said medical scanning apparatus configured to perform scans using triggered magnetic resonance imaging data acquisition, wherein the computing device is configured to provide the medical scanning apparatus with triggering times for triggered magnetic resonance imaging data acquisition.

One of the main ideas underlying the present invention is to provide a computing device which implements artificial intelligence tools in order to generate a prediction of the respiratory cycles of a patient, thereby achieving a more accurate determination of the times where the data acquisition performed by a scanning machine should take place in order to optimize the reduction of respiratory motion in free-breathing scans.

The device as described above allows for a simple implementation of a computer-implemented method comprising a number of steps. In one step, real-time data from the respiratory cycles of a patient are obtained. Based on this data, future predictions of the respiratory cycles (or breathing cycles) of the same patient are generated with the help of an artificial intelligence entity. The predicted breathing cycles are used to calculate the triggering times, located around the end-expiratory or end-inspiratory points of the predicted breathing cycles. The so determined triggering times can then be used by a medical scanning apparatus to produce scans with an improved reduction of respiratory-associated motion.

One advantage of the present invention is that the determination of the triggering times is based on the future respiratory cycles of a patient predicted by a deep learning artificial neural network. This prediction can therefore be systematically enhanced by improving the training of the artificial neural network. This ability to learn and improve the predictions is particularly useful when dealing with irregular respiratory patterns and offers a substantial improvement over conventional heuristic algorithms.

A further advantage of the present invention is that the determination of the triggering times can be adjusted closer to the end-expiratory or end-inspiratory points, thus facilitating scans with much reduced influence of the respiratory motion.

Advantageous embodiments and further developments follow from the dependent claims as well as from the description of the different preferred embodiments illustrated in the accompanying figures.

According to some embodiments, refinements, or variants of embodiments, the triggering module implements a trigger algorithm, which is configured to calculate at least one triggering time for each predicted respiratory cycle based on an extracted maximum and/or minimum amplitude values of the predicted respiratory cycles.

The trigger algorithm is configured to process the predictions generated by the artificial intelligence entity and locate the maximum (end-inspiratory) and/or minimum (end-expiratory) points in the breathing amplitudes. The end-expiratory point is typically the best option, since it is the point where the reduction of respiratory motion is easier to achieve. However, the triggering times can also be computed around the end-inspiratory point. In some embodiments, it is also possible to combine triggering times close to the end-expiratory and close to the end-inspiratory point, in order to reduce the data-acquisition time and thereby the exposure time of the patient.

The calculation of the triggering times depends on the configuration of the algorithm. For instance, the algorithm can calculate the triggering times based on splitting a determined acquisition time symmetrically around the predicted end-expiratory points.

According to some embodiments, refinements, or variants of embodiments, the artificial intelligence entity of the computation module is a deep learning network, in particular a multilayer perceptron network.

Advanced deep learning networks are especially suited to deal with complex nonlinear problems of supervised learning in image classification, where a large amount of data is available and the extraction of patterns is demanding. Given the relative degree of regularity of the respiratory cycles, which are oscillations that can be approximated with a continuous function, a multilayer perceptron (MLP) is a good compromise to achieve an accurate description while optimizing the amount of needed data both for training and for the predictions of the respiratory cycles. MLPs have the capacity to learn non-linear models and also to learn in real-time, which is a desired feature for the present invention.

According to some embodiments, refinements, or variants of embodiments, the deep learning network is trained with baseline data and/or trained with patient-specific data of the patient.

The MLP can be trained with baseline data from a respiratory surrogate signal coming from a belt, a cushion, a respiratory sensor, a pilot tone or any combination thereof. This data can be used as ground truth for respiratory traces, and can be taken from existing databases, e.g., from the k-space center of acquired radial volumetric breath-hold examination (r-VIBE) data.

In a preferred embodiment, and in order to improve the learning, this generic baseline training data can be complemented with patient-specific data, i.e. data from the respiratory cycles of the patient under exploration.

According to some embodiments, refinements, or variants of embodiments, the triggering module comprises a configuration unit, configured to set the data acquisition time.

In a default setting, the data acquisition time can be taken to be a predetermined constant value based on previous experience or adapted to the prescriptions of the scanning machine. However, depending on the patient and the current physical condition of the patient (e.g., being in a state of rest or having anxiety), the respiratory cycles can be shorter or longer in time than average. Correspondingly, the respiratory amplitude can also be affected. The configuration unit permits to adjust the data-acquisition time of the scans in order to optimize the triggering times. This adjustment of the data-acquisition time can be done manually, by a user, or automatically, e.g. based on the duration of the predicted respiratory cycles.

According to some embodiments, refinements, or variants of embodiments, the data-acquisition time is adapted to provide amplitude-symmetric triggered imaging data acquisitions. This can be automatically implemented, e.g. by monitoring the time that it takes between the trigger point and the end-expiratory point on the current respiratory cycle and releasing updated data-acquisition times on the fly to the scanning machine.

According to some embodiments, refinements, or variants of embodiments, the triggering module comprises a threshold unit, configured to set an amplitude threshold between 5% and 30% of a difference between the average minimum and the average maximum amplitude values of the obtained respiratory cycles.

In these embodiments, this amplitude threshold is not dynamic, i.e. it does not evolve with time, as in some of the conventional algorithms. The amplitude threshold can be set, e.g., to provide an upper amplitude limit, above which triggering times would be too far away from the end-expiratory point. It can also avoid data acquisition in cases where the end-expiratory points have amplitudes larger than usual, which indicates that such end-expiratory points are anomalous.

According to some embodiments, refinements, or variants of embodiments, the computing device further comprises a rating module, configured to generate a quality rating value for each acquired triggered imaging data acquisition, based on their time-wise symmetry around the respective minimum and/or maximum amplitude values and/or based on the difference between the minimum amplitude value and/or the maximum amplitude value and the amplitude value of the corresponding triggering time.

In these embodiments, the rating module provides an indication of the quality of the different data acquisitions, which can (i) indicate to the operator of the MR machine whether extra scans are needed; and/or (ii) provide valuable information to an expert before analyzing or printing the different scans. Based on the rating, the expert can, e.g., discard the worse scans and concentrate on the best ones.

The indication of the rating module is based on how well the data acquisition was centered around the end-expiratory point and also based on the difference in amplitude between the triggering point and the end-expiratory point. Both parameters are indicators of the degree of respiratory-associated motion that was in place when each scan took place.

According to some embodiments, refinements, or variants of embodiments, the rating module comprises a graphical user interface, configured to output a plot comprising an information about the triggering times, the corresponding data-acquisition times, and the amplitude of the respiratory cycle of the patient.

In other words, the triggering times and data-acquisition times can be seen (e.g. superimposed) on the actual respiratory pattern of the patient, e.g., as the data acquisition is taking place. In some embodiments, this graphical representation can be combined with the information associated to the rating. This makes the information more intuitive to the operator or expert that has to deal with the scans.

The graphical user interface can either be installed in a physical apparatus (e.g., on a scanning machine) or on a server or on a cloud computing platform.

According to some embodiments, refinements, or variants of embodiments, the rating module further comprises a tolerance unit, configured to separate, based on a tolerance threshold of the rating values, the acquired triggered imaging data acquisitions into two groups, wherein the tolerance threshold is set by a user.

In these embodiments, the parameters used in the rating of the scans can be employed as variables of a tolerance function. The tolerance function can also contain other parameters as variables, for instance the blurriness of the scans.

The tolerance threshold separates satisfactory scans from unsatisfactory ones, e.g. based on generic medical prescriptions or based on the criteria of an expert. The tolerance threshold can also depend on the particular organ or medical application that the scans will be used for and can therefore be changed based thereupon.

In some embodiments, the tolerance threshold can be monitored and updated through a command line interface, a menu-driven user interface, or a touch user interface.

According to some embodiments, refinements, or variants of embodiments, the triggering module further comprises an optimization unit, configured to optimize the trigger algorithm with an artificial intelligence entity and/or to import an optimized trigger algorithm from another computing device and/or to export the optimized trigger algorithm to another computing device.

The artificial intelligence entity can be any deep reinforcement learning network adapted to optimize the trigger algorithm of the invention. Other ways to improve the trigger algorithm involve importing more sophisticated (e.g. optimized with deep reinforcement learning) trigger algorithms from other computing devices or exporting the more sophisticated trigger algorithms to other computing devices. This network of trigger algorithms can make sure that in a hospital or clinic, all the MRI machines operate with the most sophisticated (i.e. the most trained) trigger algorithm. In case the hospital or clinic is part of a consortium, this guarantees that the different branches of the consortium can share their trigger algorithms, thereby opening the possibility of their having homogeneous equipment standards.

Although here, in the foregoing and also in the following, some functions are described as being performed by modules or units, it shall be understood that this does not necessarily mean that such modules or units are provided as entities separate from one another. In cases where one or more modules or units are provided as software, the modules or units may be implemented by program code sections or program code snippets, which may be distinct from one another, but which may also be interwoven or integrated into one another.

Similarly, in cases where one or more modules or units are provided as hardware, the functions of one or more modules or units may be provided by one and the same hardware component, or the functions of several modules or units may be distributed over several hardware components, which need not necessarily correspond to the modules or units. Thus, any apparatus, system, method and so on which exhibits all of the features and functions ascribed to a specific module or unit shall be understood to comprise, or implement, said module or said unit. In particular, it is a possibility that all modules or units are implemented by program code executed by the computing device, for example a server or a cloud computing platform.

The above embodiments and implementations can be combined with each other as desired, as far as this is reasonable.

Further scope of the applicability of the present method and device will become apparent from the following figures, detailed description and claims. However, it should be understood that the detailed description and specific examples, while indicating preferred embodiments of the invention, are given by way of illustration only, since various changes and modifications within the spirit and scope of the invention will become apparent to those skilled in the art.

Aspects of the present disclosure will be better understood with reference to the following figures.

In the figures:
Fig. 1 is a schematic depiction of a computing device for triggered imaging data acquisition in medical scans according to an embodiment of the present invention;
Fig. 2 is a block diagram showing an exemplary embodiment of a computer-implemented method for triggered imaging data acquisition corresponding to the present invention;
Fig. 3 is a schematic illustration of the operation of a conventional trigger algorithm, according to the state of the art;
Fig. 4 shows two plots, where data from a respiratory pattern is compared with the prediction generated employing a computing device according to an embodiment of the present invention;
Fig. 5 shows a comparison between the performance of a conventional algorithm for generating triggering times and the performance of a computing device according to an embodiment of the present invention;
Fig. 6 shows a comparison between the performance of a conventional algorithm for generating triggering times and the performance of a computing device according to an embodiment of the present invention, when the respiratory pattern is irregular;
Fig. 7 shows an example that illustrates the transfer of optimized trigger algorithms between different computing devices according to an embodiment of the present invention;
Fig. 8 shows a medical scanning system, comprising the computing device according to an embodiment of the present invention, and a medical scanning apparatus;
Fig. 9 is a schematic block diagram illustrating a computer program product according to an embodiment of the third aspect of the present invention; and
Fig. 10 is a schematic block diagram illustrating a non-transitory computer-readable data storage medium according to an embodiment of the fourth aspect of the present invention.

Parts in the different figures that correspond to the same elements have been indicated with the same reference numerals.

The components in the drawings are not necessarily to scale, emphasis being placed instead upon clearly illustrating the principles of the present disclosure. Likewise, certain components can be shown in generalized or schematic form in the interest of clarity and conciseness. In some instances, well-known structures and devices are shown in block diagram form in order to avoid obscuring the concepts of the present invention.

The numeration of the steps in the methods are meant to ease their description. They do not necessarily imply a certain ordering of the steps. In particular, several steps may be performed concurrently.

The detailed description includes specific details for the purpose of providing a thorough understanding of the present invention. However, it will be apparent to those skilled in the art that the present invention may be practised without these specific details.

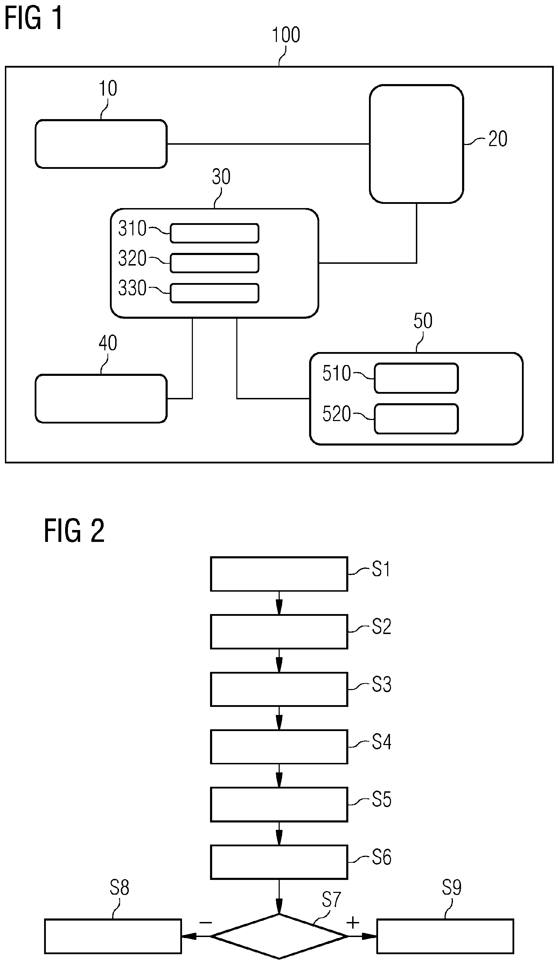

Fig. 1 shows a schematic depiction of a computing device 100 for triggered imaging data acquisition in medical scans according to an embodiment of the present invention.

The computing device 100 depicted in Fig. 1 comprises an input data interface 10, a computation module 20, a triggering module 30, an output data interface 40 and a rating module 50.

The input data interface 10 is configured to obtain real-time data of the respiratory cycles of a patient. This information can be acquired from a number of sensors, to which the computing device 100 is connected, wired or wireless or with a combination thereof.

The computation module 20 is configured to implement an artificial intelligence entity with which a prediction of the future respiratory cycles of the patient based on the obtained real-time data is generated.

The artificial intelligence entity can be any artificial neural network, most preferably advanced deep learning networks adapted to deal with complex nonlinear problems of supervised learning in image classification. For instance, a multilayer perceptron (MLP) with between 1 and 5 hidden layers can achieve an accurate prediction of the respiratory pattern of the patient using a reasonable amount of data.

The MLP can be trained with baseline data from a respiratory surrogate signal coming from a belt, a cushion, a respiratory sensor, a pilot tone or any combination thereof. This data is used as ground truth for respiratory traces and can be taken from different existing databases. For the embodiments presented in this invention, data from the k-space center of acquired radial volumetric breath-hold examination (r-VIBE) was used.

The triggering module 30 is configured to determine triggering times for the predicted respiratory cycles. In some embodiments, a trigger algorithm is deployed, which is adapted to determine the minimum of the predicted respiratory cycles (if the scans are to be taken around the end-expiratory time point), the maximum of the predicted respiratory cycles (if the scans are to be taken around the end-inspiratory time point), or both (if both time points are to be considered).

The triggering times are then calculated, which indicate the beginning of the data acquisition to be performed by the scanning machine. The calculation of the triggering times depends on the configuration of the trigger algorithm. For instance, the trigger algorithm can calculate the triggering times based on splitting a fixed data acquisition time symmetrically around the predicted end-expiratory points.

In some embodiments, such as the one depicted in Fig. 1, the triggering module 30 comprises a configuration unit 310, configured to set the data acquisition time.

In a default setting, the data acquisition time can be taken to be a predetermined constant value based either on previous experience with similar scans or adapted to the prescriptions of the scanning machine. However, depending on the patient and the current physical condition of the patient (e.g., being in a state of rest or having anxiety), the respiratory cycles can be very different. With the configuration unit 310 the data-acquisition time of the scans can be modified in order to optimize the triggering times. This adjustment of the data-acquisition time can be done manually, e.g. by the operator of the scanning machine, or automatically, e.g. based on the duration of the predicted respiratory cycles. In some embodiments, the data-acquisition time is computed such that it is (amplitude-) symmetric around the end-expiratory points. This can be achieved, e.g., by monitoring the time that it takes between the trigger point and the end-expiratory point corresponding to the current respiratory cycle and releasing updated data-acquisition times on the fly to the scanning machine. In these embodiments, the data-acquisition time is not a fixed variable but a dynamical one that depends on the characteristics of each breathing cycle.

In the embodiments depicted in Fig. 1, the triggering module 30 further comprises a threshold unit 320, which is configured to set an amplitude threshold value for the triggering times. In some preferred embodiments, the threshold is set between 5% and 30% of the difference between the average minima and average maxima of the obtained respiratory cycles. This trigger threshold can be set, e.g., in order to avoid triggering times too far away from the end-expiratory point.

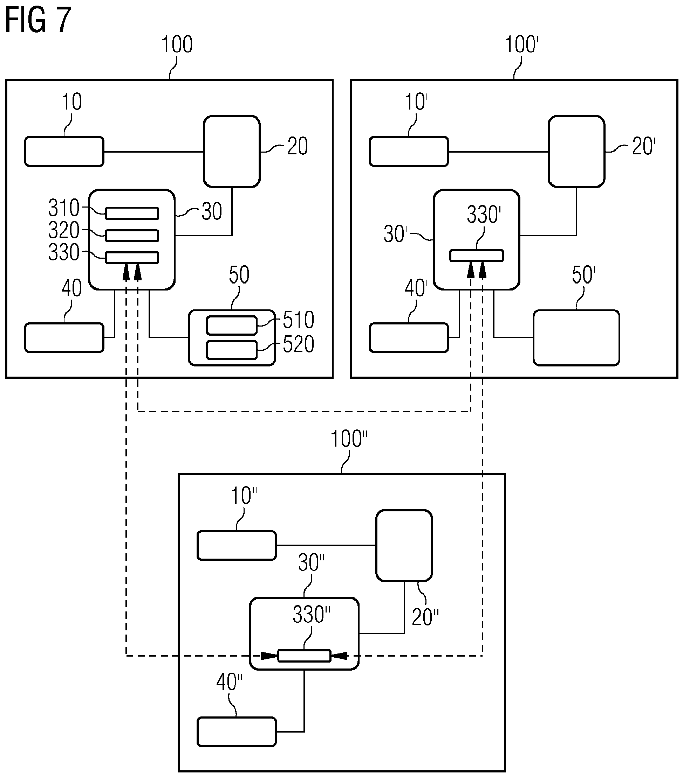

In the embodiment of Fig. 1, the triggering module 30 further comprises an optimization unit 330, which is configured to improve the trigger algorithm. In one embodiment, this can be done with the help of an artificial intelligence entity, e.g., any deep reinforcement learning network adapted to optimize the trigger algorithm of the invention. In other embodiments, the optimization unit 330 can exchange trigger algorithms among similar computing devices (see, e.g., the description of Fig. 7).

The output data interface 40 is configured to output the triggering times determined by the triggering module for the triggered imaging data acquisition to be performed by a scanning machine. The output data interface 40 is connected wired or wireless with the scanning machine, to which it delivers the corresponding set of instructions.

In the embodiment of Fig. 1, the computing device 100 further comprises a rating module 50, which is configured to rate the scans acquired by the scanning apparatus according to the triggering times. This rating is based on the symmetry (in amplitude or in time) around the end-expiratory point and/or the difference in amplitude value between the triggering time and the end-expiratory point. In some embodiments, the rating module 50 comprises a graphical unit 510, which is configured to provide a visualization of the triggering times and data-acquisition times superimposed on the actual respiratory pattern of the patient. In some embodiments, this graphical representation can be combined with the information associated to the rating. This makes the information more intuitive to the operator or expert that has to deal with the scans.

The graphical representation can be visualized in a graphical user interface, which can either be installed in a physical apparatus (e.g., on the scanning machine) or it can be accessed or retrieved from a server or a cloud computing platform.

In the embodiment of Fig. 1, the rating module 50 further comprises a tolerance unit 520, which separates the scans based on a tolerance threshold. A tolerance function is built, which employs as variables the parameters used in the scan rating.

The tolerance threshold separates satisfactory from unsatisfactory ones, and it is based either on generic medical prescriptions or based on the criteria of an expert. This generically depends on the organs to be scanned. The tolerance threshold can be monitored and updated through a command line interface, a menu-driven user interface, or a touch user interface.

The computing device 100 of Fig. 1 can be implemented partially or completely in a scanning machine, e.g. an MRI machine.

Fig. 2 shows a block diagram of an exemplary embodiment of a computer-implemented method for triggered imaging data acquisition corresponding to the present invention, preferably to be performed with the computing device 100 depicted in Fig. 1. The method comprises a number of steps.

In a step S1, real-time data of the breathing cycles of a patient are obtained. This data is used by an artificial intelligence entity, preferably a multilayer perceptron (MLP) network, to generate, in a step S2, a prediction for the breathing pattern of the patient in a future time.

Based on the prediction, in a step S3, triggering times are determined for the upcoming respiratory cycle(s) of the patient. In a step S4, these triggering times are output and subsequently used by a scanning machine to perform the triggered scans.

In the embodiment described in Fig. 2, a number of steps S5-S9 are also provided. In the computing device depicted in Fig. 1, these steps can be performed with the rating module 50.

In a step S5, each of the acquired triggered imaging data acquisition is rated, i.e. it is given a quality rating value, which can be based on the degree of time-wise symmetry around the respective minimum and/or maximum amplitude values of the respiratory cycles. It can also be based on the difference between the minimum amplitude value (or the maximum amplitude value), and the amplitude value of the corresponding triggering time.

In a step S6, the actual breathing pattern of the patient is plotted in a graphical user interface 510, together with the different triggering times and the data-acquisition times. In some embodiments, the plot also contains the rating values of the different triggered data acquisitions.

In a step S7, a tolerance threshold (a specific rating value) is set, which is used to separate the different triggered data acquisitions based on their quality. If the rating value of a triggered data acquisition is below the tolerance threshold (case marked with the symbol - in Fig. 2), then in a step S8 the triggered data acquisition is considered unsatisfactory. If, on the contrary, the rating value of a triggered data acquisition is above the tolerance threshold (case marked with the symbol + in Fig. 2), in a step S9 the triggered data acquisition is considered satisfactory. This separation of the acquired scans according to their quality can be used by a medical expert to sort out the most significant scans.

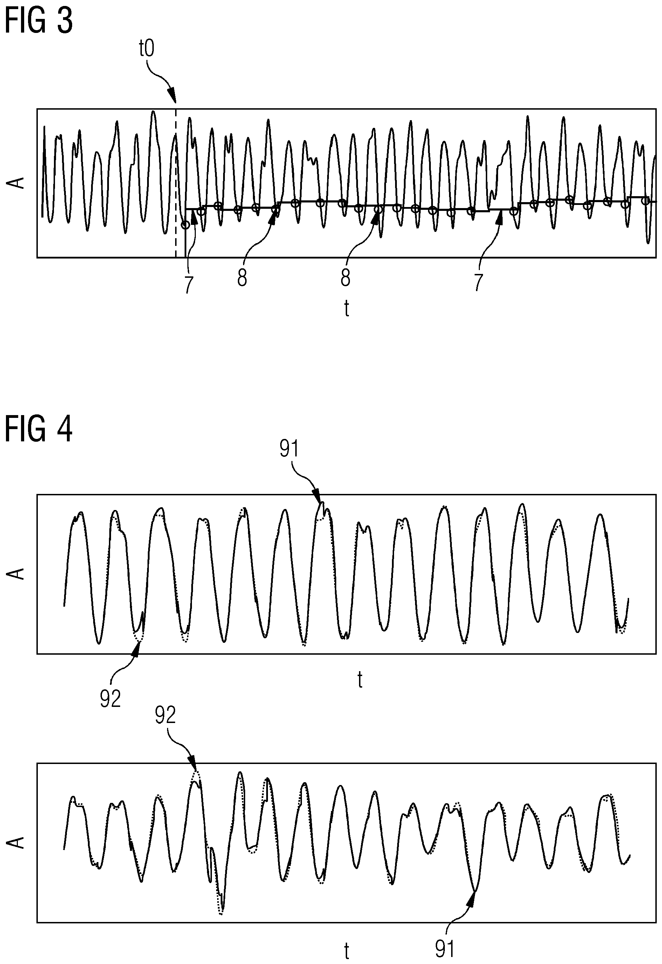

Fig. 3 shows a schematic illustration of the operation of a conventional trigger algorithm, according to the state of the art. The plot describes the amplitude A of a succession of respiratory cycles as a function of time t. Both the amplitude A and the time t are given in arbitrary units. After an initial phase, at time t0 the trigger algorithm calculates an amplitude threshold 7, based on the 20% of the difference of the average maximum and minimum amplitude values of the respiratory cycles during the initial phase (before the time t0). This value of the amplitude threshold 7 gets updated after each respiratory cycle by calculating the average minimum and maximum of the three most recent cycles.

If the amplitude A of the respiratory signal goes under the threshold 7, a triggering time 8 is generated and a medical scanning apparatus (not shown in Fig. 3) makes a data acquisition. The data-acquisition time takes a predetermined constant value for all the triggering times 8.

Fig. 4 shows two plots, where data from a respiratory pattern is compared with the prediction generated employing a computing device 100 according to an embodiment of the present invention.

After a learning phase (not shown in Fig. 4), the amplitude A of a reference respiratory pattern 91 is predicted using a multilayer perceptron (MLP) with one hidden layer. The MLP was trained with 24 minutes of respiratory signals taken from the k-space center of acquired radial volumetric breath-hold examination (r-VIBE) data. The respiratory signals were split into 72 dataset batches of 20 seconds each.

In order to generate the predicted respiratory pattern 92, the computing module 30 was set to make predictions up to one second ahead in the future. In other words, the MLP network was using the reference respiratory cycles 91 as learning input to make predictions of the respiratory pattern one second later.

The predicted respiratory pattern 92 and the reference respiratory pattern 91 are shown superimposed in both plots. In the upper plot, discrepancies between the two curves arise mostly near the maxima (the end-inspiratory points). The minima (the end-expiratory points) are more regular and very well predicted by the MLP. This generic feature is also observed in the lower plot, which describes a less regular reference respiratory pattern 91.

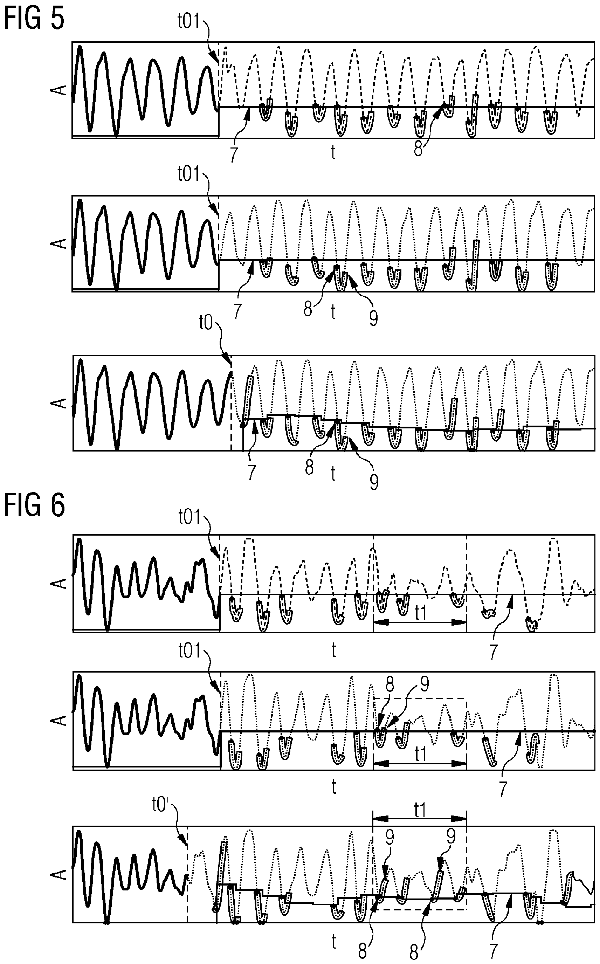

Fig. 5 shows a comparison between the performance of a conventional algorithm for generating triggering times 8 and the performance of a computing device according to an embodiment of the present invention.

The upper plot of Fig. 5 shows a reference respiratory pattern up to a time t01, followed by a predicted respiratory pattern. The MLP is the same as in Fig. 4 and has undergone its training with the same baseline data. The MLP uses the reference respiratory pattern up to t01 as additional learning data.

After the time t01, an amplitude threshold 7 is calculated by the threshold unit 320 (see Fig. 1), by taking 20% of the difference of the average maximum and minimum amplitude values of the reference respiratory pattern before the time t01. This amplitude threshold 7 stays constant over time.

The trigger algorithm of the triggering module 30 calculates the triggering times 8 corresponding to each predicted respiratory cycle based on the extracted minima of each predicted respiratory cycle. Valid triggering times are only generated if the amplitude of the predicted respiratory pattern is sufficiently below the amplitude threshold 7. Optimal triggering times 8 are calculated in this particular example taking into account a constant data-acquisition time.

The middle plot in Fig. 5 shows the triggering times 8 and the data-acquisition times superimposed on the actual respiratory pattern. Optimally, a triggering time 8 should produce a time-wise symmetric data-acquisition time around the end-expiratory time of each respiratory cycle. It should also lay sufficiently close to the end-expiratory time while allowing a sufficient data-acquisition time for the scanning machine to correctly produce the scans.

The lower plot in Fig. 5 shows the triggering times 8 with the same data-acquisition time calculated with the conventional algorithm used in Fig. 3. The initial phase is slightly longer than t01 and finishes at t0. By comparing the middle plot with the lower plot, one can appreciate an overall improvement of the triggering times 8 when the trigger algorithm of the present invention is used. This is, for instance, illustrated by comparing the amplitude between the triggering point 8 and the end-time of data acquisition 9 between the two curves. Further optimization of the triggering times 8 in the present invention could be achieved by modifying the data-acquisition times, as has been mentioned in the foregoing when describing the different embodiments of the invention.

Fig. 6 shows a comparison between the performance of a conventional algorithm for generating triggering times 8 and the performance of a computing device according to an embodiment of the present invention, when the respiratory pattern is irregular.

As in Fig. 5, the upper and middle correspond to the present invention, while the lower plot corresponds to the conventional algorithm used in Fig. 3. The upper and middle plot of Fig. 5 show a reference respiratory pattern up to a time t01, followed by a predicted respiratory pattern. The MLP is the same as in Fig. 4 and has undergone its training with the same baseline data. The MLP uses the reference respiratory pattern up to t01 as additional learning data. The lower plot of Fig. 5 ends the initial phase at a different time t0'.

The calculation of the triggering times 8 proceeds exactly as described for the sample respiratory pattern of Fig. 5. Likewise, the data-acquisition time is kept constant.

The advantages of the present invention over conventional algorithms are more manifest when the respiratory pattern presents irregularities, as can be seen from Fig. 6. For illustration, one can concentrate on the time interval t1. On the lower plot, the triggering times 8 are too close to the end-expiratory times and the end-times 9 of the different scans are close to the upcoming maxima. This would yield bad quality scans.

On the middle plot, with the computing device 100 of the present invention, the generated triggering times 8 would yield better scans. In some cases, the trigger algorithm even skips a respiratory cycle to generate a triggering time 8 if the predicted respiratory cycle is too irregular. The present invention is therefore more robust and resilient against irregularities in the respiratory patterns.

Fig. 7 shows an example to illustrate the transfer of optimized trigger algorithms between different computing devices 100, 100', 100'' according to an embodiment of the present invention. The computing devices 100, 100', 100'' correspond to different embodiments of the invention. They all comprise an optimization unit 330, 330', 330" inside the respective triggering modules 30, 30', 30'' . Some of the optimization units 330, 330', 330" might implement an artificial intelligence entity, trained and adapted to optimize the trigger algorithm. The optimization units 330, 330', 330" are configured to exchange information, in particular the trigger algorithms.

This networking among the different computing devices 100, 100', 100'' contributes to the optimization of the trigger algorithms, allowing the medical scanning apparatuses to which the computing devices 100, 100', 100'' provide triggering times to operate with the most sophisticated (i.e. the most trained) trigger algorithm of the network.

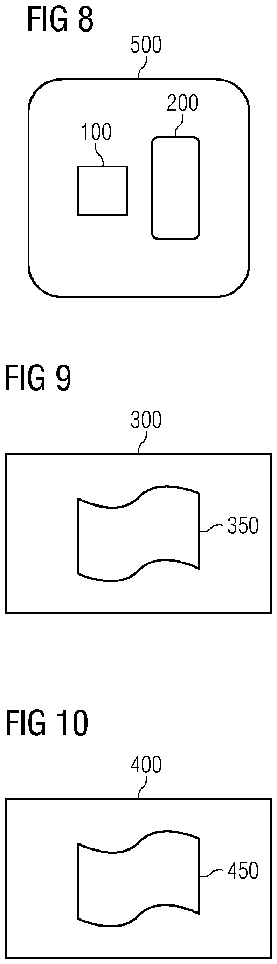

Fig. 8 shows a medical scanning system 500, comprising the computing device 100 according to an embodiment of the present invention, and a medical scanning apparatus 200.

The medical scanning apparatus 200 can be a magnetic resonance scanner, configured to perform scans using triggered magnetic resonance imaging data acquisition. The computing device 100 provides the medical scanning apparatus 200 with triggering times, such that it can perform the triggered scans.

In some embodiments, the computing device 100 or parts thereof are integrated in the medical scanning apparatus 200. In particular, the input data interface 10 (see Fig. 1), which gathers data from a number of sensors, and/or the output data interface 40 (see Fig. 1), which provides the scanning apparatus 200 with information about the triggering times, can be naturally integrated in the scanning apparatus 200. Parts of the rating module 50 (see Fig. 1), in particular the graphical unit 510 or the tolerance unit 520, which are also useful for the operation of the scanning apparatus 200, can also advantageously be integrated in it. The same applies to the configuration unit 310 of the triggering module 30. Parts of the computing device 100 that are not integrated in the scanning apparatus 200 can be connected to it.

Fig. 9 shows a schematic block diagram illustrating a computer program product 300 according to an embodiment of the third aspect of the present invention. The computer program product 300 comprises executable program code 350 configured to, when executed, perform the method according to any embodiment of the second aspect of the present invention, in particular as it has been described with respect to the preceding figures.

Fig. 10 shows a schematic block diagram illustrating a non-transitory computer-readable data storage medium 400 according to an embodiment of the fourth aspect of the present invention. The data storage medium 400 comprises executable program code 450 configured to, when executed, perform the method according to any embodiment of the second aspect of the present invention, in particular as it has been described with respect to the preceding figures.

The non-transient computer-readable data storage medium may comprise, or consist of, any type of computer memory, in particular semiconductor memory such as a solid-state memory. The data storage medium may also comprise, or consist of, a CD, a DVD, a Blu-Ray-Disc, an USB memory stick or the like. The previous description of the disclosed embodiments are merely examples of possible implementations, which are provided to enable any person skilled in the art to make or use the present invention. Various variations and modifications of these embodiments will be readily apparent to those skilled in the art, and the generic principles defined herein may be applied to other embodiments without departing from the spirit or scope of the present disclosure. Thus, the present invention is not intended to be limited to the embodiments shown herein but it is to be accorded the widest scope consistent with the principles and novel features disclosed herein. Therefore, the present invention is not to be limited except in accordance with the following claims.

## Claims

1. A computing device (100, 100', 100") for providing triggering times (8) to a medical scanning apparatus (200) adapted to perform triggered imaging data acquisition, said computing device (100, 100', 100'') comprising:
an input data interface (10, 10', 10"), configured to obtain real-time data of respiratory cycles of a patient;
a computation module (20, 20', 20''), configured to implement an artificial intelligence entity, which is adapted, in particular trained and adapted, to generate a prediction of a number of respiratory cycles of the patient based on the obtained real-time data;
a triggering module (30, 30', 30''), configured to determine triggering times (8) corresponding to the predicted respiratory cycles; and
an output data interface (40, 40', 40''), configured to output the determined triggering times (8) to the medical scanning apparatus (200).

2. The computing device (100, 100', 100") according to claim 1, wherein the triggering module (30, 30', 30") implements a trigger algorithm, which is configured to calculate at least one triggering time (8) for each predicted respiratory cycle based on an extracted maximum and/or minimum amplitude values of the predicted respiratory cycles.

3. The computing device (100, 100', 100") according to any of the preceding claims, wherein the artificial intelligence entity of the computation module (20, 20', 20'') is a deep learning network, in particular a multilayer perceptron network.

4. The computing device (100, 100', 100") according to claim 3, wherein the deep learning network is trained with baseline data and/or trained with patient-specific data of the patient.

5. The computing device (100) according to any of the preceding claims, wherein the triggering module (30) comprises a configuration unit (310), configured to set the data acquisition time.

6. The computing device (100) according to claim 5, wherein the data-acquisition time is adapted to provide amplitude-symmetric triggered imaging data acquisitions.

7. The computing device (100) according to any of the preceding claims, wherein the triggering module (30) comprises a threshold unit (320), configured to set an amplitude threshold (7) between 5% and 30% of a difference between the average minimum and the average maximum amplitude values of the obtained respiratory cycles.

8. The computing device (100, 100') according to any of the preceding claims, further comprising a rating module (50, 50'), configured to generate a quality rating value for each acquired triggered imaging data acquisition, based on their time-wise symmetry around the respective minimum and/or maximum amplitude values and/or based on the difference between the minimum amplitude value and/or the maximum amplitude value and the amplitude value of the corresponding triggering time (8).

9. The computing device (100) according to claim 8, wherein the rating module (50) comprises a graphical user interface (510), configured to output a plot comprising an information about the triggering times (8), the corresponding data-acquisition times, and the amplitude (A) of the respiratory cycle of the patient.

10. The computing device (100) according to claim 8, wherein the rating module (50) further comprises a tolerance unit (520), configured to separate, based on a tolerance threshold of the rating values, the acquired triggered imaging data acquisitions into two groups, wherein the tolerance threshold is set by a user.

11. A system of computing devices (100, 100', 100") according to any of the preceding claims, wherein the triggering module (30, 30', 30") further comprises an optimization unit (330, 330', 330''), configured to optimize the trigger algorithm with an artificial intelligence entity and/or to import an optimized trigger algorithm from another computing device (100, 100', 100'') and/or to export the optimized trigger algorithm to another computing device (100, 100', 100").

12. A computer-implemented method for providing triggering times (8) to a medical scanning apparatus (200) adapted to perform triggered imaging data acquisition, preferably to be implemented with the computing device (100, 100', 100") according to any of the claims 1 to 11, comprising the following steps:
obtaining (S1) real-time data of respiratory cycles of a patient;
generating (S2), using an artificial intelligence entity, a prediction of a number of respiratory cycles of the patient based on the obtained real-time data;
determining (S3) triggering times (8) corresponding to the predicted respiratory cycles; and
outputting (S4) the triggering times (8) to the medical scanning apparatus (200).

13. A medical scanning system (500), comprising the computing device (100, 100', 100") according to claims 1 to 11, and a medical scanning apparatus (200), said medical scanning apparatus configured to perform scans using triggered magnetic resonance imaging data acquisition, wherein the computing device (100, 100', 100") is configured to provide the medical scanning apparatus (200) with triggering times (8) for triggered magnetic resonance imaging data acquisition.

14. A computer program product (300) comprising executable program code (350), which is configured, when executed, to perform the computer-implemented method according to claim 12.

15. A non-transient computer-readable data storage medium (400) comprising executable program code (450), which is configured, when executed, to perform the computer-implemented method according to claim 12.
